# EUROPEAN PATENT APPLICATION

(11) **EP 3 120 873 A1**
(43) Date of publication of application: **25.01.2017**
(21) Application number: 15178013.7
(22) Date of filing: 23.07.2015
(51) Int. Cl.: A61L 9/12

(54) **TWO PHASE LIQUID EVAPORATOR DEVICE**

(71) Applicant: Eurvest, 1400 Nivelles (BE)
(72) Inventor: BONNEVILLE, Julien, 59730 Briastre (FR); BERTIGNON, Estelle, 7000 MONS (BE); LUCIANI, Alain, 13390 AURIOL (FR)
(74) Representative: August & Debouzy avocats

(57) **Abstract**

The present invention is directed to a two-phase evaporator device comprising one recipient comprising a lower aqueous phase and an upper oily phase and at least one hydrophobic wick of a first material M1 and at least one amphiphilic wick of a second material M2, which both extend from the interior of the recipient in contact with the two phases to the exterior where the evaporation area thereof is located.

## Description

### FIELD OF THE INVENTION

The present invention relates generally to the field of a device which dispenses fragrance or other liquids. More specifically, this device uses a two-phase liquid composition.

### TECHNICAL BACKGROUND

Several solutions exist for evaporator devices, especially home fragrance diffuser; such as reed or synthetic wick diffusers, plugged devices or spray.

An existing solution involves conventional reed fragrance diffusers. This system uses multiple thin reeds and a fragrance oil composition. It diffuses mainly through absorption of the fragrance oil into the wood. Once the reeds are saturated, the fragrance oil desorbs from the reeds and diffuses into the surrounding atmosphere, delivering the fragrance. This system presents the drawbacks to dry easily and to reduce evaporation of the fragrance.

Another existing solution involves oil-in-water diffusers. Emulsifying the fragrance in an aqueous phase involves the use of surfactants. However, surfactants are non-volatile molecules and their presence in the composition reduces evaporation. In addition, realizing a surfactant based emulsion limits the proportion of fragrance incorporable in the aqueous phase. The diffusion of fragrance in moist environment enhances the perception of the perfume, so it is desirable to use a two-phase evaporator device.

In the document EP 1 847 175, the evaporator device dispenses a two phases composition. Two wicks, composed of the same material are used to diffuse each of the two phases outside the recipient. One of the wicks is layered from the lower extremity to the stopper cover of the recipient preventing contact with the other liquid phase. The addition of an impermeable layer increase the manufacturing cost of the process and limits the flexibility of the device.

In the document WO 2004/032983, the evaporator device comprises a container, with one embodiment involving multiple compartments for holding an oil-based liquid and a water-based liquid separately and another embodiment with one compartment comprising a mixture of the two liquids. At least one wick for delivering the two liquids with different properties is described and includes two sections; the first section is made of hydrophobic materials and the second section is made of hydrophilic materials. The two solutions exhibit either the surfactant limitation or the use of a more complex container.

Therefore, there is a need for a surfactant free, layer free improved home fragrance two phases diffuser to overcome these and other problems.

### SUMMARY OF THE INVENTION

This has been achieved with the instant invention.

A first aspect of the invention is concerned with a two-phase evaporator device comprising one recipient comprising a lower aqueous phase and an upper oily phase and at least one hydrophobic wick of a first material M1 and at least one amphiphilic wick of a second material M2, which both extend from the interior of the recipient in contact with the two phases to the exterior where the evaporation area thereof is located.

According to one embodiment of the invention, the amphiphilic material can be chosen from lignocellulosic materials.

According to one embodiment of the invention, the amphiphilic material is wood, preferably rattan.

According to one embodiment of the invention, the hydrophobic material can be chosen from the group consisting of polymer.

According to one embodiment of the invention, the hydrophobic material is polyester.

According to one embodiment of the invention, the ratio of the oily phase to the aqueous phase is from 10:90 to 90:10, preferably from 75:25 to 20:80 more preferably from 60:40 to 25:75.

According to one embodiment of the invention, the ratio of the oily phase to the aqueous phase is constant over time.

According to one embodiment of the invention, the diffusion rate of the oily phase to the aqueous phase is constant.

According to one embodiment of the invention, none of the wick is impermeably layered.

According to one embodiment of the invention, the sections of the wicks are variable along their length.

According to one embodiment of the invention, the surface of the evaporation area is comprised between 300 mm² and 5000 mm², preferably between 600 mm² and 2500 mm².

According to one embodiment of the invention, the volume of the recipient is comprised between 10 mL and 1000 mL, preferably between 30mL and 500 mL, and even more preferably between 50 mL and 100 mL.

According to one embodiment of the invention, the recipient is closed or open.

According to one embodiment of the invention, the phases are surfactant free.

According to one embodiment of the invention, the number of wicks is comprised between 2 and 16, usually between 4 and 8.

### DRAWINGS

Figure 1 shows a sectional view of a two-phase evaporator device according to the invention which has wicks of different material.
Figure 2a shows a diagram representing the phases volume evolution according to the time in days.
Figure 2b shows a graph representing the total weight loss of the phases according to the time in days.

### DESCRIPTION OF EMBODIMENTS OF THE INVENTION

In general, the present invention relates to an evaporator device containing two immiscible liquids in which at least two wicks are dipped and diffuse passively the fragrance included into one of the two liquids.

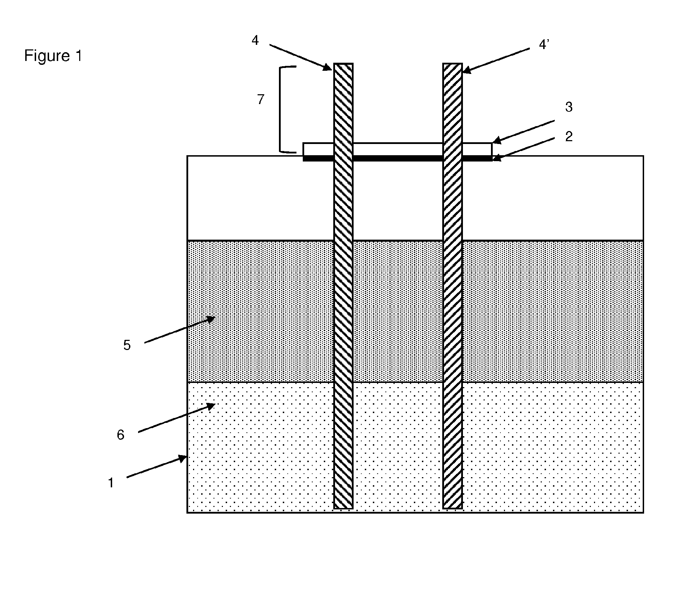

According to Figure 1, the evaporator device comprises a recipient 1 which contains a liquid. The recipient has a single opening 2. The opening 2 is sealed by a lid 3; it is also possible not to have a lid. At least two porous or capillary wicks 4, 4' extend from the recipient 1 through the opening 2. Two wicks are represented but it is possible to use more. None of the wick has an impermeable layer in the embodiment shown. The wicks 4, 4' are of different material and characteristics. The wicks 4, 4' extend from the exterior of the recipient 1 into the liquids. The fragrance composition 5, located in the upper level, i.e. the oily phase, of the bi-phase liquid, is absorbed by one of the wick and travels along its length via capillary or porosity action. The aqueous composition 6, located in the lower level of the bi-phase liquid, also travels along the second wick length; leading to a moist environment of the evaporation area 7. The fragrance composition 5 is subsequently diffused from the external surfaces of the wick 4, 4' into the ambient environment.

The wicks are also referred as reeds or sticks or strips and these terms are used interchangeably to design the same element. These are made of different materials and possess different characteristics. Preferably, the wicks do not have an impermeable layer. By "wick" it is understood a piece of material that conveys liquid by capillary action or porous action. It can be a reed, a stick or a strip. The selection or prevention of contact with one of the liquids into the wick through its lower extremity can be achieve thanks to the difference in material of each wick and their respective affinity for a particular liquid. This lead to a simultaneous and constant diffusion of the two immiscible liquids in the recipient.

The recipient is typically formed from a plastic material, for example polyolefins such as PE/PP, polyester such as PET, or a glass material. The recipient is preferably translucent so that the level of the two-phase liquid inside can be seen through its walls. The recipient usually contains volumes comprise between 10 mL and 1000 mL, preferably between 30 mL and 500 mL, and even more preferably between 50 mL and 100 mL. It can have different geometry, it is usually round-shaped and rectangular-shaped but other shapes are possible such as cubic, pyramidal, cylinder or conical shaped.

The number of wicks of the present invention is comprised between 2 and 16, usually between 4 and 8.

The wicks are in contact with the two immiscible liquids contained in the recipient.

The "evaporation area" can be defined as the area of the wick that is in contact with the atmosphere above the lid.

In the invention, one wick carries one phase of the liquid, another wick carries the other phase of the liquid. A first wick substantially only carries the liquid situated at the upper level, i.e. the oily phase, up to the evaporation area of the wick. The first wick has a particular affinity with this liquid, here the oil. It is made, for example, of plastic polymers, usually polyester. The second wick only carries the liquid situated at the lower level, here the aqueous phase, up to the evaporation area of the wick. The second wick has a particular affinity with this liquid, here water. It is made, for example, of wood, usually rattan.

The applicant has surprisingly discovered that the wicks, and especially the second wick, have different behaviors depending on the phase(s) they are dipped in. Indeed, when the second wick is dipped in a one phase aqueous composition, the second wick only diffuses the aqueous phase. When the second wick is dipped in a one phase oil composition, the second wick only diffuses the oil composition. Surprisingly, when the second wick is dipped in a bi-phase bi-layer composition with one aqueous layer or phase and one oily layer or phase, (i.e. there is no mixture of the two phases); the second wick diffuses substantially only the aqueous composition. The second wick which is amphiphilic then appears to have a modified behavior in the bi-phase composition. The same behavior also can be seen for the first wick, albeit to a much lesser extent.

The materials of the wick are selected based on various criteria. The configuration of the wick is taken into account, as well as the capillarity or porosity required for such product. The present wicks are typically not layered which bring even more modularity in contrast to the other existing solutions.

The surface of the wick is not necessarily constant through its length. By "surface" it is understood the outer or the topmost boundary of an object. It usually has a cylindrical geometry. In other embodiment according to the present invention, the wick can have other geometrical section, for example rectangle, triangle or square. This difference of section will lead to a difference in diffusion depending of the phases. Indeed, a greater diffusion surface provides more evaporation of the liquid. Other characteristics of the evaporator device can also modulate the diffusion of the fragrance such as the difference of material chosen for the wick or the composition of the two liquids.

The wick usually have a length comprises between 10 cm and 50 cm, usually between 15 cm and 25 cm in order to adapt well to the recipient in which it is dipped and also to enhance the diffusion performance of the present invention.

The two liquids contained in the recipient of the present invention are immiscible; meaning that the two liquids are incapable of being mixed to form a homogeneous substance. As a result, this bi-phase composition has specific physicochemical properties.

A first phase, situated at the lower level of the composition because its density is higher, is an aqueous phase intended to humidify the atmosphere for a better olfactory performance. The aqueous phase typically comprises water as solvent, pH buffer such as the couple citric acid, trisodium citrate and at least a preservative, for example isothiazolinone. The aqueous phase may also contains dyes to color to the aqueous phase.

A second phase, situated at the upper level of the composition, is an oily phase dedicated to the fragrance diffusion. This oily phase is at the upper level because its density is lower than then density of the lower phase. The oily phase usually comprises a volatile silicon oil as solvent, for example cyclopentasiloxane, fragrance and dyes. By "fragrance" it is understood a mixture of solvent and fragrant essential oils extracted from flowers, spices, etc., or made synthetically, used especially to impart a pleasant long-lasting scent.

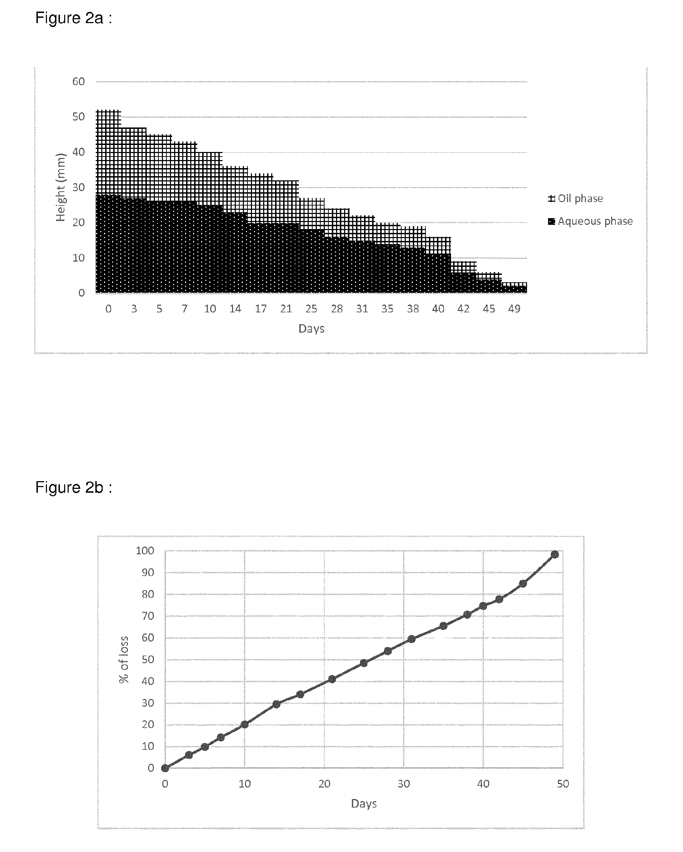

The fragrance, situated in the organic phase, is absorbed by a first wick and diffuses along its length. A second wick will absorb preferentially the lower level liquid, which is the aqueous phase. The second wick will be saturated in lower level liquid and the upper level liquid will then be impeded from rising up to this wick. One advantage of the present invention is that the fact to use two different types of wick allows the simultaneous diffusion of the two phases. The diffusion of the fragrance and the diffusion of water (which allows moisturizing the evaporation area) are simultaneous. This simultaneity enhances the perception of the fragrance in the atmosphere. According to Figure 2, a simultaneous diffusion is achieved with an evaporation rate proportional to the volume of each phase, the ratio of the two phases remaining substantially constant over time.

The composition of the present invention is surfactant free. The main advantage of this characteristic is that the composition is not foaming; this avoids any risk of clogging the pores of the wicks. The ratio of the oily phase to the aqueous phase can vary broadly and is generally from 10:90 to 90:10, preferably from 75:25 to 20:80 more preferably from 60:40 to 25:75.

The composition of the bi-phases formulation for the following examples is detailed in Table 1.

**Table 1**

| Oil phase | | Aqueous phase | |
|---|---|---|---|
| Compound | Percentage | Compound | Percentage |
| Silicon oil | QSP 100% | Osmosed water | QSP 100% |
| Fragrance | 10% | Preservative | 0.10% |
| Dye | < 1000 ppm | Dye | < 1000 ppm |

The amphiphilic character of the wood wick under a bi-phase composition can be exemplified in the below Table 2. Indeed, the different coefficients of diffusion K of several compositions are shown in Table 2 and may be used to deduce the properties imparted by each wick. In the table the coefficient K is expressed in g/day*cm².

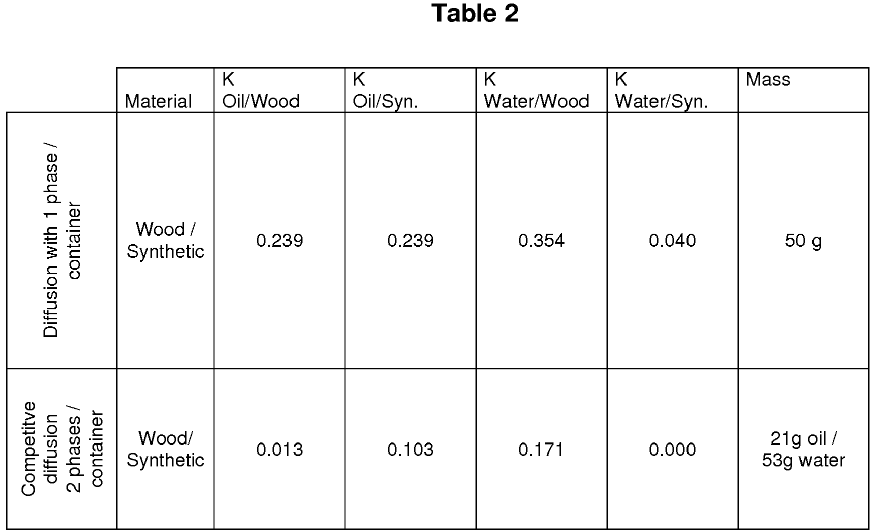

Different tests have been realized at 20°C and under 55% of hygrometry. A number of 6 wicks have been used, either 6 synthetic wicks or 6 wood wicks. The phase ratio, in volume, used for the tests is of 30% of oil phase of 70% of aqueous phase. The value of the coefficient of diffusion is independent from the phase ratio.

During the tests in the bi-phase composition, the phases are diffused sequentially according to the nature of the wick:
- with the synthetic wicks, the diffusion of the oil phase is observed for the oily phase, and a very low diffusion of the aqueous phase is observed as well. When the same wicks are dipped into the two layers composition, the oily diffusion remains, albeit divided by about 2 (the value goes from 0.239 to 0.103) while aqueous diffusion is substantially suppressed (the value goes from 0.04 down to 0).
- with the wood wicks, the diffusion of the oil phase is observed for the oily phase, and the diffusion of the aqueous phase is observed as well, at a high level. When the same wicks are dipped into the two layers composition, the aqueous diffusion remains albeit divided by about 2 (the value goes from 0.354 to 0.171) while the oily diffusion is substantially reduced, divided by a factor of about 20 (the value goes from 0.239 to 0.013).

This surprising behavior allows the use of a simple container with one compartment, two distinct immiscible phases with no surfactant while at the same time avoiding the use of layered wicks.

To obtain the results shown in Figures 2, different tests have been realized at 20°C and under 55% of hygrometry. A number of 6 wicks have been used, 3 synthetic wicks and 3 wood wicks. The phase ratio, in volume, used for the tests is of 50% of oil phase of 50% of aqueous phase. The recipient has the same section throughout its height and contains 100 mL of bi-phase formulation.

According to Figure 2a, a phase volume evolution for a 50:50 oil/water ratio composition has been recorded.

According to Figure 2b, a simultaneous diffusion is achieved with an evaporation rate proportional to the volume of each phase, the ratio of the two phases remaining substantially constant over time. Figure 2b exhibits a linear behavior with a constant weight loss over time.

## Claims

1. A two-phase evaporator device comprising
- one recipient comprising a lower aqueous phase and an upper oily phase,
- at least one hydrophobic wick of a first material M1 and at least one amphiphilic wick of a second material M2, which both extend from the interior of the recipient in contact with the two phases to the exterior where the evaporation area thereof is located.

2. The two-phase evaporator device according to claim 1 wherein the amphiphilic material can be chosen from lignocellulosic materials.

3. The two-phase evaporator device according to claim 1 or 2, wherein the amphiphilic material is wood, preferably rattan.

4. The two-phase evaporator device according to anyone of claims 1 to 3, wherein the hydrophobic material can be chosen from the group consisting of polymer.

5. The two-phase evaporator device according to anyone of claims 1 to 3, wherein the hydrophobic material is polyester.

6. The two-phase evaporator device according to anyone of claims 1 to 4, wherein the ratio of the oily phase to the aqueous phase is from 10:90 to 90:10, preferably from 75:25 to 20:80 more preferably from 60:40 to 25:75.

7. The two-phase evaporator device according to anyone of claims 1 to 6, wherein the ratio of the oily phase to the aqueous phase is constant over time.

8. The two-phase evaporator device according to anyone of claims 1 to 7, wherein the diffusion rate of the oily phase to the aqueous phase is constant.

9. The two-phase evaporator device according to anyone of claims 1 to 8, wherein none of the wick is impermeably layered.

10. The two-phase evaporator device according to anyone of claims 1 to 9, wherein the sections of the wicks are variable along their length.

11. The two-phase evaporator device according to anyone of claims 1 to 10, wherein the surface of the evaporation area is comprised between 300 mm² and 5000 mm², preferably between 600 mm² and 2500 mm².

12. The two-phase evaporator device according to anyone of claims 1 to 11, wherein the volume of the recipient is comprised between 10 mL and 1000 mL, preferably between 30 mL and 500 mL, and even more preferably between 50 mL and 100 mL.

13. The two-phase evaporator device according to anyone of claims 1 to 12, wherein the recipient is closed or open.

14. The two-phase evaporator device according to anyone of claims 1 to 13, wherein the phases are surfactant free.

15. The two-phase evaporator device according to anyone of claims 1 to 14, wherein the number of wicks is comprised between 2 and 16, usually between 4 and 8.
